# EUROPÄISCHE PATENTSCHRIFT

(11) **EP 2 623 029 B1**
(45) Veröffentlichungstag und Bekanntmachung des Hinweises auf die Patenterteilung: **25.11.2020**
(21) Anmeldenummer: 13152041.3
(22) Anmeldetag: 21.01.2013
(51) Int. Cl.: A61B 5/055

(54) **Hilfsvorrichtung zur Untersuchung eines Hüftgelenks**
Auxiliary device for examining a hip joint
Dispositif d'aide destiné à l'examen d'une articulation de la hanche

(30) Priorität: 02.02.2012 AT 500222012
(43) Veröffentlichungstag der Anmeldung: 07.08.2013
(73) Patentinhaber: Menges, Manfred, 4076 St. Marienkirchen an der Polsenz (AT)
(72) Erfinder: Menges, Manfred, 4076 St. Marienkirchen an der Polsenz (AT)
(74) Vertreter: Hübscher & Partner Patentanwälte GmbH

(56) Entgegenhaltungen:
- DE-A1- 10 310 389
- DE-U1- 8 909 993
- US-A- 5 542 423
- US-A- 5 991 651
- US-A1- 2003 131 855

## Beschreibung

Die Erfindung bezieht sich auf eine Hilfsvorrichtung zur Untersuchung eines Hüftgelenks mit einem Magnetresonanztomographen.

Dem Stand der Technik entsprechende Hilfsvorrichtungen für Magnetresonanztomographen sind zum Beispiel aus US2003/0131855A1, US5991651 A und DE10310389A1 bekannt.

Zur Vorbereitung von Hüftgelenksoperationen ist die Kenntnis des Knorpelzustands im Bereich des Kopfes des Oberschenkelknochens und der Gelenkpfanne bzw. des Zustands des Kopfbands sowie die Beschaffenheit der Gelenkslippe eine wesentliche Voraussetzung, um unter Bezugnahme auf die örtliche Ausdehnung eines festgestellten Gelenkschadens beispielsweise die weitere operative Vorgangsweise und Planung festlegen zu können. Um sich mit Hilfe eines Magnetresonanztomographen ein gutes Bild über die örtliche Erstreckung und Größe eines allfälligen Gelenkschadens machen zu können, wäre es vorteilhaft, das Hüftgelenk mit einem Kontrastmittel zu füllen und während der Untersuchung einer Zugbelastung zu unterwerfen. Bestehende Geräte zur Magnetresonanztomographie lassen allerdings keine Zugbelastung des zu untersuchenden Hüftgelenks zu.

Der Erfindung liegt somit die Aufgabe zugrunde, eine bei herkömmlichen Magnetresonanztomographen einsetzbare Hilfsvorrichtung zu schaffen, mit deren Hilfe eine Zugbelastung des zu untersuchenden Hüftgelenks sichergestellt werden kann, ohne die Magnetresonanztomographie zu beeinträchtigen.

Die Erfindung löst die gestellte Aufgabe durch eine Hilfsvorrichtung nach Anspruch 1.

Zufolge dieser Maßnahmen kann das Bein des zu untersuchenden Hüftgelenks über das Zugmittel mit einer vorgegebenen, vorzugsweise einstellbaren Zugkraft belastet werden, während sich das andere Bein an der Fußstütze des Gestells abstützt. Dies bedeutet zunächst, dass durch die Abstützung des nicht durch eine Zugkraft belasteten Beins am Gestell einem Kippen des Beckens und damit einem dadurch bedingten Verlust der Zugbelastung des zu untersuchenden Hüftgelenks entgegengewirkt werden kann. Damit wird eine einfache Voraussetzung für eine Hilfsvorrichtung geschaffen, die unabhängig vom jeweils zum Einsatz kommenden Magnetresonanztomographen für eine gleichmäßige Zugbelastung des Hüftgelenks mit der Wirkung sorgt, dass aufgrund des belastungsbedingten Auseinanderziehens der Gelenkteile insbesondere bei einer vorausgehenden Kontrastmittelfüllung das Hüftgelenk besser eingesehen und damit zuverlässiger untersucht werden kann.

Obwohl die Zugbelastung des Zugmittels gegenüber dem Gestell konstruktiv auf unterschiedliche Art, beispielsweise mit Hilfe von Federn oder Zylindern, erreicht werden kann, ergeben sich besonders einfache Konstruktionsbedingungen, wenn das am Bein des zu untersuchenden Hüftgelenks befestigbare Zugmittel um eine Umlenkrolle des Gestells geführt und an ein Belastungsgewicht angeschlossen wird. Aufgrund der Gewichtsbelastung des an einem Bein eines Patienten befestigten Zugmittels und der Abstützung des anderen Beins am Gestell wird das zu untersuchende Hüftgelenk während der Untersuchung unter einer entsprechenden Zugbelastung gehalten.

Damit die auf das Zugmittel aufgebrachte Zugkraft vorteilhaft auf das zu belastende Bein übertragen werden kann, kann das Zugmittel mit Hilfe eines geeigneten Schuhs am Bein des zu untersuchenden Hüftgelenks befestigt werden. Mit einem solchen Schuh kann eine weitgehend gleichmäßige und für den Patienten schonende Beinbelastung sichergestellt werden.

Damit für das Gestell ein ausreichender Verstellweg bis zum Magnetresonanztomographen berücksichtigt werden kann, ist die Fußabstützung unter Umständen dem fahrbaren Gestell in einem entsprechenden Abstand vorzulagern. Zu diesem Zweck empfiehlt es sich, die Fußabstützung mit einer Führungsstange zu versehen, die im Gestell in Richtung der Längsachse des Patienten verstellbar gelagert ist, um die Lage der Fußabstützung gegenüber dem Gestell und damit die Lage des Gestells gegenüber dem Magnetresonanztomographen an die jeweiligen Raumverhältnisse anpassen zu können. Dies bedeutet, dass mittels der Hilfsvorrichtung in einfacher Weise unterschiedliche Tischhöhen und -längen berücksichtigt werden können.

Um die Hilfsvorrichtung auf einfache Art zur Untersuchung eines linken oder rechten Hüftgelenks umrüsten zu können, kann das Gestell zwei bezüglich des Zugmittels symmetrisch angeordnete Führungslager für die mit einer Fußabstützung versehene Führungsstange aufweisen. Mit dieser Maßnahme wird es möglich, die Fußabstützung je nach Bedarf für ein linkes oder rechtes Bein einzusetzen, indem die Führungsstange für die Fußabstützung in das jeweilige der beiden beidseits des Zugmittels vorgesehenen Führungslager eingesetzt wird. Das Zugmittel bleibt gegenüber dem zu belastenden Bein ausgerichtet.

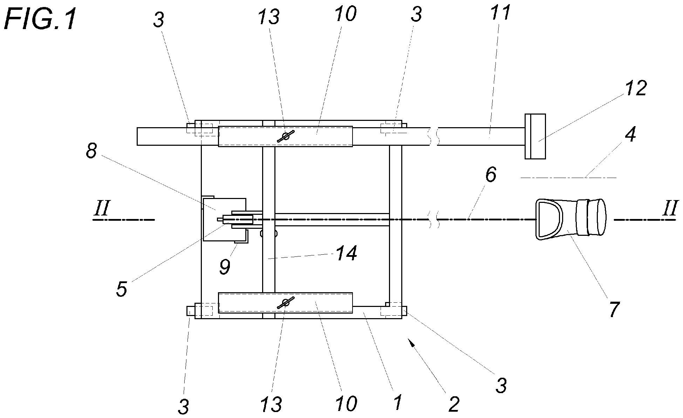

In der Zeichnung ist der Erfindungsgegenstand beispielsweise dargestellt. Es zeigen
- Fig. 1: eine erfindungsgemäße Hilfsvorrichtung zur Untersuchung eines Hüftgelenks mit einem Magnetresonanztomographen in einer vereinfachten Draufsicht,

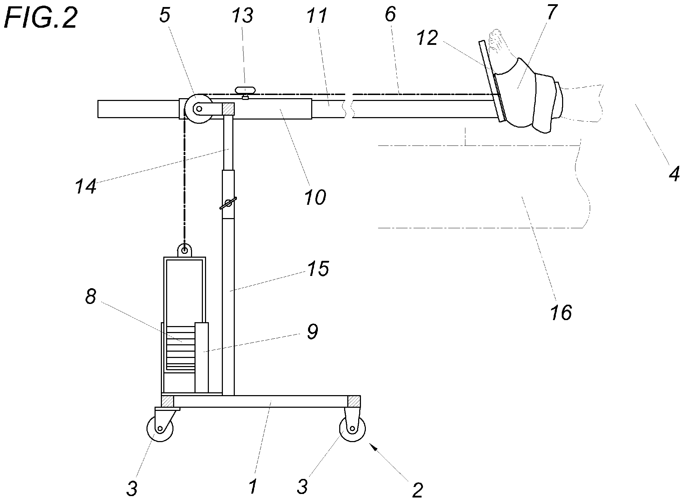

- Fig. 2: diese Hilfsvorrichtung in einem Schnitt nach der Linie II-II der Fig. 1 und

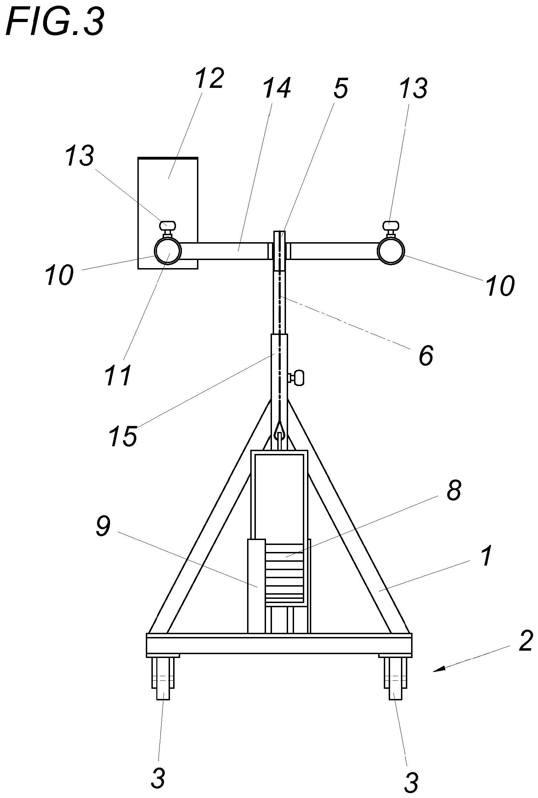

- Fig. 3: die Hilfsvorrichtung in einer stirnseitigen Ansicht.

Die dargestellte Hilfsvorrichtung weist ein Gestell 1 mit einem Fahrwerk 2 auf, dessen Räder 3 eine Positionierung des Gestells bezüglich der Längsachse 4 eines Patienten erlauben. Auf dem Gestell 1 ist eine Umlenkrolle 5 für ein Zugmittel 6, vorzugsweise ein Seil, gelagert, das ein in Richtung der Längsachse 4 verlaufendes Trum mit einem angeschlossenen Schuh 7 aufweist, mit dessen Hilfe das Zugmittel 6 am Bein eines Patienten befestigt werden kann. Am anderen Ende des Zugmittels 6 ist ein Belastungsgewicht 8 angehängt, das in einer Führung 9 der Höhe nach verschiebbar gehalten wird und sich vorzugsweise aus einem Satz von Einzelgewichten zusammensetzt, um eine Anpassung der Zugbelastung des zu untersuchenden Hüftgelenks an die jeweiligen anatomischen Gegebenheiten und das Gewicht des Patienten zu ermöglichen.

Symmetrisch zu beiden Seiten des Zugmittels 6 sind zwei Führungslager 10 zur wahlweisen Aufnahme einer Führungsstange 11 im Gestell 1 vorgesehen, die an ihrem vorderen Ende eine Fußabstützung 12 trägt. Zur Einstellung der über das jeweilige Führungslager 10 vorkragenden Länge der Führungsstange 11 ist die Führungsstange 11 in den Führungslagern 10 längsverstellbar gehalten und kann in der gewählten Verschiebestellung mit Hilfe von Klemmschrauben 13 festgestellt werden. Zur Höhenanpassung der Fußabstützung 12 und des in der Gebrauchsstellung zur Führungsstange 11 im Wesentlichen parallelen Trums des Zugmittels 6 ist der die Umlenkrolle 5 und die Führungslager 10 für die Führungsstange 11 umfassende Teil 14 des Gestells 1 gegenüber dem Fahrwerk 2 der Höhe nach verlagerbar. Im dargestellten Ausführungsbeispiel ist zu diesem Zweck der Teil 14 teleskopartig in einem Tragrohr 15 des Gestells 1 verstellbar gelagert.

Zur Untersuchung eines Hüftgelenks wird der auf einem Tisch 16 des Geräts zur Magnetresonanztomographie ruhende Patient mit dem Bein des zu untersuchenden Hüftgelenks, im dargestellten Ausführungsbeispiel also mit dem linken Bein, an das Zugmittel 6 angeschlossen, indem der Schuh 7 um den Fuß des linken Beins gelegt und geschlossen wird. Das andere, im dargestellten Fall rechte Bein stützt sich an der Fußstütze 12 ab. Aufgrund der Zugbelastung des Zugmittels 6 über das Belastungsgewicht 8, wird der Patient gegen die Fußabstützung 12 gezogen, sodass sich der Patient über das auf der Fußabstützung 12 aufruhende Bein gegenüber dem Gestell 1 abstützen kann, was für die Beckenlage bei der einseitigen Zugbelastung des anderen Beins von wesentlicher Bedeutung ist. Wegen der Zugbelastung des einen Beins und der Abstützung über das andere Bein bedarf es an sich keiner zugfesten Verbindung zwischen der Fußabstützung 12 und dem abgestützten Fuß, obwohl es vorteilhaft sein kann, eine entsprechende Verbindung, beispielsweise durch einen den Fuß im Vorfußbereich umspannenden Ristriemen, herzustellen.

Durch die Zugbelastung des linken Beins und der gleichzeitigen Abstützung des rechten Beins kann einem Kippen des Beckens entgegengewirkt werden, was das Aufbringen einer durch das Belastungsgewicht 8 vorgegebenen Zugbelastung auf das Hüftgelenk vereinfacht. Das dadurch bedingte Auseinanderziehen der zusammenwirkenden Gelenkteile lässt in Verbindung mit einer Kontrastmittelfüllung einen besseren Einblick in das Hüftgelenk und damit ein genaueres Untersuchungsergebnis zu.

Zur Untersuchung des rechten Hüftgelenks ist die Hilfsvorrichtung umzurüsten, wobei die Führungsstange 11 mit der Fußabstützung 12 in das linke Führungslager 10 eingesetzt wird. Sind an linke und rechte Füße angepasste Schuhe 7 vorgesehen, so ist auch der Schuh 7 zu wechseln, bevor der Patient an die Hilfsvorrichtung angeschlossen werden kann.

## Patentansprüche

1. Hilfsvorrichtung zur Magnetresonanztomographie-Untersuchung eines Hüftgelenks eines auf dem Tisch eines Magnetresonanztomographen ruhenden Patienten, wobei die Hilfsvorrichtung ein Gestell (1) hat, das ein mit einer vorgegebenen Zugkraft in Richtung der Längsachse (4) des Patienten belastbares Zugmittel (6) sowie eine in Richtung der Längsachse (4) des Patienten wirksame Fußabstützung (12) aufweist, **dadurch gekennzeichnet, dass** das Zugmittel (6) zur Belastung des Beins des zu untersuchenden Hüftgelenks mit einer Zugkraft an diesem Bein befestigbar ist während die Fußabstützung (12) das andere Bein abstützt.

2. Hilfsvorrichtung nach Anspruch 1, **dadurch gekennzeichnet, dass** das am Bein des zu untersuchenden Hüftgelenks befestigbare Zugmittel (6) um eine Umlenkrolle (5) des Gestells (1) geführt und an ein Belastungsgewicht (8) angeschlossen ist.

3. Hilfsvorrichtung nach Anspruch 1 oder 2, **dadurch gekennzeichnet, dass** das Zugmittel (6) mit Hilfe eines Schuhs (7) am Bein des zu untersuchenden Hüftgelenks befestigbar ist.

4. Hilfsvorrichtung nach einem der Ansprüche 1 bis 3, **dadurch gekennzeichnet, dass** die Fußabstützung (12) eine Führungsstange (11) aufweist, die im Gestell (1) in Richtung der Längsachse (4) des Patienten verstellbar gelagert ist.

5. Hilfsvorrichtung nach Anspruch 4, **dadurch gekennzeichnet, dass** das Gestell (1) zwei bezüglich des Zugmittels (6) symmetrisch angeordnete Führungslager (10) für die mit einer Fußabstützung (12) versehene Führungsstange (11) aufweist.

## Claims

1. Auxiliary device for examining, by means of magnetic resonance imaging, a hip joint of a patient lying on the table of a magnetic resonance imaging system, wherein the auxiliary device has a frame (1) which comprises a traction means (6), which can be loaded with a specified tensile force in the direction of the longitudinal axis (4) of the patient, and a foot support (12) which is effective in the direction of the longitudinal axis (4) of the patient, **characterised in that** the traction means (6) for loading the leg of the hip joint to be examined with a tensile force can be fastened to this leg while the foot support (12) supports the other leg.

2. Auxiliary device as claimed in claim 1, **characterised in that** the traction means (6) which can be fastened to the leg of the hip joint to be examined is guided around a deflection roller (5) of the frame (1) and is connected to a loading weight (8).

3. Auxiliary device as claimed in claim 1 or 2, **characterised in that** the traction means (6) can be fastened to the leg of the hip joint to be examined with the aid of a shoe (7).

4. Auxiliary device as claimed in any one of claims 1 to 3, **characterised in that** the foot support (12) comprises a guide rod (11) which is mounted in the frame (1) so as to be adjustable in the direction of the longitudinal axis (4) of the patient.

5. Auxiliary device as claimed in claim 4, **characterised in that** the frame (1) comprises two guide bearings (10), which are arranged symmetrically in relation to the traction means (6), for the guide rod (11) provided with a foot support (12).

## Revendications

1. Dispositif auxiliaire pour réaliser un examen d'imagerie par résonance magnétique d'une hanche d'un patient couché sur la table d'un système d'imagerie par résonnance magnétique, ce dispositif auxiliaire possédant un châssis (1) qui comporte un moyen de traction (6) pouvant être chargé avec une force de traction prédéfinie dans la direction de l'axe longitudinal (4) du patient ainsi qu'un repose-pieds (12) agissant dans la direction de l'axe longitudinal (4) du patient, **caractérisé en ce que** le moyen de traction (6) pour charger la jambe de la hanche à examiner peut être fixé à cette jambe avec une force de traction tandis que le repose-pieds (12) soutient l'autre jambe.

2. Dispositif auxiliaire selon la revendication 1, **caractérisé en ce que** le moyen de traction (6) pouvant être fixé à la jambe de la hanche à examiner est guidé autour d'une poulie de renvoi (5) du châssis (1) et attaché à une masse de lestage (8).

3. Dispositif auxiliaire selon la revendication 1 ou 2, **caractérisé en ce que** le moyen de traction (6) peut être fixé à la jambe de la hanche à examiner à l'aide d'une chaussure (7).

4. Dispositif auxiliaire selon une des revendications 1 à 3, **caractérisé en ce que** le repose-pieds (12) présente une tige de guidage (11) qui est placée de manière réglable dans le châssis (1) dans la direction de l'axe longitudinal (4) du patient.

5. Dispositif auxiliaire selon la revendication 4, **caractérisé en ce que** le châssis (1) possède deux paliers de guidage (10) disposés symétriquement par rapport au moyen de traction (6) pour la tige de guidage (11) dotée d'un repose-pieds (12).
